# EUROPÄISCHE PATENTSCHRIFT

(11) **EP 1 958 570 B1**
(45) Veröffentlichungstag und Bekanntmachung des Hinweises auf die Patenterteilung: **12.01.2011**
(21) Anmeldenummer: 07003209.9
(22) Anmeldetag: 15.02.2007
(51) Int. Cl.: A61B 8/06

(54) **Verfahren zur Darstellung anatomischer Patientenstrukturen im interessierenden Bereich eines Bilderfassungsgeräts**
Method for illustrating anatomical patient structures of the section in question on an imaging device
Procédé de représentation de structures anatomiques de patients dans la zone pertinente d'un appareil de saisie d'image

(43) Veröffentlichungstag der Anmeldung: 20.08.2008
(73) Patentinhaber: BrainLAB AG, 85622 Feldkirchen (DE)
(72) Erfinder: Leidel, Martin, 83714 Miesbach (DE); Vollmer, Fritz, 80469 München (DE); Thiemann, Ingmar, 80686 München (DE)
(74) Vertreter: Schwabe - Sandmair - Marx

(56) Entgegenhaltungen:
- DE-A1-102004 038 610
- US-A- 5 538 004
- US-B1- 6 193 660
- US-B1- 6 368 277
- US-B1- 6 663 568

## Beschreibung

Die Erfindung betrifft ein Verfahren zur Darstellung einer anatomischen Patientenstruktur im interessierenden Bereich eines beweglichen Bilderfassungsgeräts. Insbesondere betrifft sie das technische Gebiet der Ultraschall-Bilderfassung, speziell der Strömungsgeschwindigkeitsmessung in Gefäßen (Blutgefäßen) durch Doppler-Ultraschall-Bilderfassung.

Die oben angesprochenen Ultraschall-Doppler-Strömungsbilder werden normalerweise in einem kleineren Teil eines Standard-B-Modus-Ultraschallbildes gezeigt. Der Benutzer muss einen interessierenden Bereich (ROI = region of interest) relativ zu den Bildkoordinaten, also relativ zum Koordinatensystem des Ultraschallkopfes, definieren, wobei dann in diesem Bereich die Strömungsinformationen farbkodiert dargestellt werden.

Wenn die Ultraschall-Sonde (Kopf) bewegt wird, behält der interessierende Bereich seine Position innerhalb des Bildes, weil er in Relation zum Sonden-Koordinatensystem festgelegt wird. Wegen der Bewegung kann aber die darzustellende anatomische Struktur (beispielsweise ein Blutgefäß) sich aus dem interessierenden Bereich entfernen, und der Verwender muss diesen interessierenden Bereich dann manuell neu festlegen (beispielsweise durch eine Eingabe an der Hardware/Softwareunterstützung des Ultraschallgerätes).

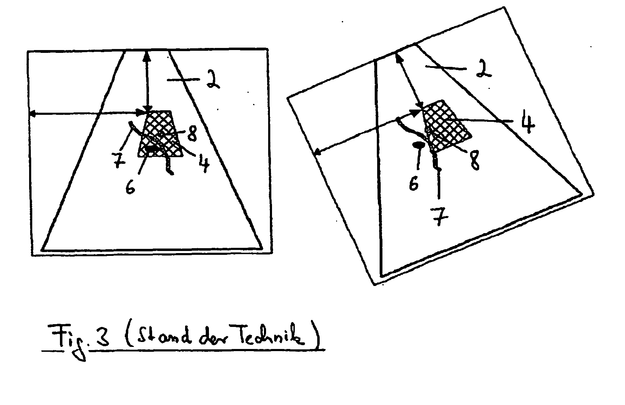

Diese Situation lässt sich anhand der beiliegenden Figur 3 verdeutlichen. Im linken Bild zeigt die Figur 3 eine erste Ultraschallaufnahme. In der Ultraschall-Erfassungsebene 2 liegen drei hier beispielhaft dargestellte Gefäße 6, 7 und 8, wobei das Gefäß 7 entlang seines Verlaufs und die Gefäße 6 und 8 im Querschnitt abgebildet werden. Der Benutzer legt nun manuell einen interessierenden Bereich fest, der sich in der Umgebung der Gefäße 6, 7, 8 befindet, und die Schnittfläche des interessierenden Bereiches mit der Erfassungsebene 2 ist in den beiden Darstellungen der Figur 3 kreuzschraffiert dargestellt und mit dem Bezugszeichen 4 versehen worden.

Wenn nun das Ultraschallgerät bewegt wird, bewegt sich auch der ihm gegenüber positionell festgelegte interessierende Bereich, wo die Strömung gut dargestellt werden kann, mit. Es kann eine Situation entstehen, wie sie im rechten Bild der Figur 3 aufgezeigt ist, wo die Schnittfläche 4 des interessierenden Bereichs mit der Erfassungsebene 2 nicht mehr wie gewünscht den gesamten Bereich der Strukturen 6, 7 und 8 abdeckt. Im vorliegenden Fall würde lediglich das Gefäß 8 noch wenigstens teilweise im interessierenden Bereich, d.h. in der Schnittfläche 4 liegen, aber die Gefäße 6 und 7 könnten nicht mehr strömungsmäßig erfasst werden.

Um wieder eine Strömungserfassung zu ermöglichen, muss der interessierende Bereich manuell in seiner Position gegenüber dem Ultraschallgerät verändert bzw. verschoben werden. Die Beobachtung des Patienten wird dadurch nachteiligerweise unterbrochen und die Handhabung wird aufwändig. Um dieses Problem zu vermeiden, wurden in der Vergangenheit oft relativ große interessierende Bereiche festgelegt, damit die darzustellenden Strukturen sich auch nach einer Bewegung noch in diesen Bereichen befanden. Dies hat aber den technischen Nachteil, dass die Bildwiederholungsfrequenz des Ultraschallsystems sich bei größeren interessierenden Bereichen, insbesondere bezüglich der Dopplerinformation stark verringert, so dass eine sehr langsame Bildreaktion, also ein sehr langsamer Bildaufbau in Kauf genommen werden musste. Ohne eine solche Änderung der Einstellung ist der Bewegungsspielraum des Ultraschallgeräts sehr eingeschränkt.

Aus der EP 1 041 395 B1 ist ein Verfahren zum Einstellen eines interessierenden Bereichs in einer Abbildung bekannt, wobei allerdings lediglich die Form des interessierenden Bereichs geändert wird, wenn die Tiefe oder Position verändert wird, um die Größe oder Anzahl der Abtastlinien konstant zu halten. Die US 6,193,660 schlägt vor, die Bewegung des interessierenden Bereiches aus einer Korrelation zwischen erhaltenen Bildern zu bestimmen und den interessierenden Bereich demgemäß zu verschieben. Dabei wird die Korrelation vorzugsweise auf der Basis anatomischer Merkmale oder prominenter Bildmerkmale (Kanten) errechnet.

Es ist eine Aufgabe der vorliegenden Erfindung, ein Verfahren zur Darstellung einer anatomischen Patientenstruktur im interessierenden Bereich eines beweglichen Bilderfassungsgeräts zu optimieren. Insbesondere soll die notwendige Verschiebung des interessierenden Bereichs von Hand oder durch rechenaufwändige Bildverarbeitungsmaßnahmen vermieden werden, und speziell wird angestrebt, eine gute Bildqualität und Bildwiederholungsrate über den gesamten Darstellungszeitraum und den interessierenden Bereich aufrecht zu erhalten.

Diese Aufgabe wird erfindungsgemäß durch ein Verfahren gemäß dem Patentanspruch 1 gelöst. Die Unteransprüche definieren bevorzugte Ausführungsformen der Erfindung.

Mit anderen Worten ausgedrückt, verwendet die vorliegende Erfindung also die Navigation, d.h. die Positionsbestimmung und Positionsverfolgung des Bilderfassungsgerätes dazu, den interessierenden Bereich an der richtigen Stelle zu halten bzw. zu platzieren. Navigations- bzw. Trackingsysteme sind in vielen Behandlungsumfeldem schon verfügbar, und insbesondere Ultraschallgeräte werden oft mit Navigations-Referenzeinrichtungen versehen, um ihre Bilder positionell in die bildunterstützte Chirurgie integrieren zu können. Gerade in solchen Fällen bietet die vorliegende Erfindung einen großen Vorteil, da ohnehin schon vorhandene Informationen über die Position des Bilderfassungsgerätes nun auch dazu verwendet werden können, den interessierenden Bereich des Geräts optimal zu positionieren. Wegen der Vorteile der vorliegenden Erfindung, nämlich der Möglichkeit, die zu beobachtenden Strukturen ohne manuelle Eingriffe und ohne komplizierte Bildverarbeitungsverfahren immer mit der geforderten guten Bildqualität und - information beobachten zu können, ist es aber durchaus auch denkbar, extra zum erfindungsgemäßen Zweck eine Navigation bzw. ein Tracking in einem Behandlungsumfeld zu installieren.

Die Korrelation der Daten kann sehr einfach erfolgen, weil in solchen Navigations-Umfeldern regelmäßig auch Patienten mit von ihnen vorab erstellten Bilddatensätzen (CT, MR, Röntgen, usw.) referenziert bzw. registriert sind.

An dieser Stelle wird darauf hingewiesen, dass das Bilderfassungsgerät jedwedes Bilderfassungsgerät sein kann, bei dem ein interessierender Bereich festgelegt werden kann. Die Erfindung beschränkt sich deshalb nicht nur auf Ultraschall-Bilderfassungsgeräte, sondern beispielsweise auch auf entsprechend ausgestattete Bilderfassungsgeräte wie Computertomographen, Kemspintomographen, Röntgenbild-Erfassungsgeräte und ähnliches.

Bei einer Ausführungsform der Erfindung ist der interessierende Bereich ein interessierendes Volumen im Erfassungsbereich des Bilderfassungsgeräts, und dem interessierenden Bereich wird eine definierte Position im raumfesten oder patientenfesten Koordinatensystem zugeordnet. Der interessierende Bereich des Bilderfassungsgeräts kann über dessen Softwareunterstützung vor der Benutzung (bei der präoperativen Planung), anfangs oder während der Darstellung manuell, d.h. durch einen Benutzer festgelegt werden. Gemeint ist hier eine initiale Festlegung, um einen Ausgangspunkt für den interessierenden Bereich zu fixieren.

Wenn das bewegliche Bilderfassungsgerät während der Bilddarstellung bewegt wird, kann der interessierende Bereich des Bilderfassungsgerätes, speziell durch Einstellen der Geräteparameter, entsprechend der Bewegung des Bilderfassungsgeräts nachgeführt werden.

Es besteht erfindungsgemäß auch die Möglichkeit, den interessierenden Bereich des Bilderfassungsgerätes über dessen Softwareunterstützung anfangs oder während der Darstellung automatisch und softwareseitig festzulegen, und zwar bei einem darzustellenden Abschnitt mit der Patientenstruktur.

Der darzustellende Abschnitt muss im Rahmen der vorliegenden Erfindung nicht ein stillstehender Abschnitt bei einer Patientenstruktur sein. Er kann auch während der Bilddarstellung verschoben werden, insbesondere entlang der Patientenstruktur, wobei der interessierende Bereich des Bilderfassungsgeräts dann entsprechend der Bewegung des darzustellenden Abschnitts nachgeführt wird.

Bei einer Ausführungsvariante des erfindungsgemäßen Verfahrens wird die Größe und/oder die Form des interessierenden Bereichs verändert, insbesondere an die Patientenstruktur angepasst.

Gemäß einer Ausführungsform der Erfindung wird als Bilderfassungsgerät ein Ultraschall-Bilderfassungsgerät verwendet, insbesondere ein Doppler-Ultraschallgerät zur Erfassung von Strömungseigenschaften, speziell Strömungsgeschwindigkeiten, in durchströmten Patientengefäßen, speziell Blutgefäßen. Hier besteht noch die Möglichkeit, die Winkellage des durchströmten Gefäßes zur Bilderfassungsebene des Ultraschall-Bilderfassungsgeräts aus der Schnittgeometrie des Schnittbildes des Gefäßes zu bestimmen und die ermittelten Daten über die Strömungseigenschaften (Strömungsgeschwindigkeit) entsprechend des Winkels zu korrigieren.

Die Erfindung wird im Weiteren anhand einer bevorzugten Ausführungsform und unter Bezugnahme auf die beiliegenden Zeichnungen näher erläutert. Sie kann alle hierin beschriebenen Merkmale einzeln und in jedweder sinnvollen Kombination umfassen. In den Zeichnungen zeigen:
- Figur 1: die räumliche Einordnung eines interessierenden Bereichs einer Ultraschallsonde in einem raumfesten bzw. patientenfesten Koordinatensystem;
- Figur 2: die Nachführung bzw. Verschiebung des interessierenden Bereiches nach der Bewegung des Bilderfassungsgerätes; und
- Figur 3: die Verschiebung des interessierenden Bereiches, wie sie bei Verfahren gemäß dem Stand der Technik stattfindet.

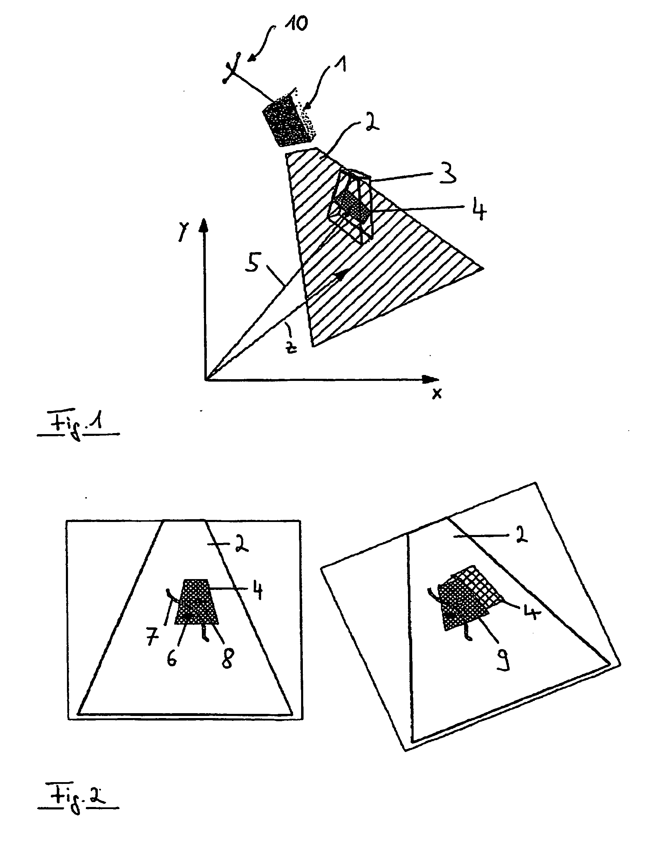

Die vorliegende Erfindung verwendet im dargestellten Ausführungsbeispiel eine navigierte Ultraschall-Integration, wobei die Positionsinformation, die mit dem Ultraschallbild verbunden ist, verwendet wird, um einen interessierenden Bereich zu definieren, welcher wiederum erfindungsgemäß im Koordinatensystem des Patienten "fixiert" wird. Die Ultraschallsonde und das Ultraschallbild sind in ihrer Ortsdarstellung kalibriert, wodurch eine genaue Zuordnung der Ultraschall-Videokoordinaten zum Patienten- oder "Welt"-Koordinatensystem möglich wird. Der interessierende Bereich kann beispielsweise ein dreidimensionaler, kastenartiger Bereich sein, wie er in Figur 1 mit dem Bezugszeichen 3 angezeigt ist. In dieser Figur 1 ist auch eine Ultraschallsonde 1 dargestellt, sowie deren Bilderfassungsebene 2. Der interessierende Bereich 3 ist ein Bereich, dessen Position anfänglich fest in Bezug auf die Ultraschallsonde 1 eingestellt ist, und bei einem Doppler-Ultraschall ist es ein Bereich, in dem beispielsweise farblich spezielle Strömungseigenschaften, wie Strömungsgeschwindigkeiten, gut wiedergegeben werden können. Die Schnittfläche des interessierenden Bereiches mit der Erfassungsebene 2 der Ultraschallsonde 1 ist kreuzschraffiert und mit dem Bezugszeichen 4 angedeutet. Die Ultraschallsonde 1 befindet sich in einem Behandlungsumfeld, welches durch ein raumfestes oder patientenfestes Koordinatensystem bestimmt wird, das in Figur 1 mit x, y und z aufgespannt ist. Der Mittelpunkt des interessierenden Patientenbereiches 3 ist in seiner Position durch den Vektor 5 im Koordinatensystem x, y, z definiert. Weil der interessierende Bereich anfänglich positionsfest gegenüber der Ultraschallsonde 1 definiert wird, bewegt er sich bei Bewegungen der Ultraschallsonde 1 im raumfesten oder patientenfesten Koordinatensystem x, y, z, bleibt aber im Koordinatensystem u, v, w der Ultraschallsonde 1 am selben Punkt. Der Sondenkopf 1 wird in einem (nicht dargestellten) Navigations- und Trackingsystem positionell geortet und verfolgt, und zwar mittels einer an ihm angebrachten Referenzanordnung 10, die im vorliegenden Fall drei Reflektorenmarker umfasst.

Durch die Navigation bzw. das Tracking der Sonde 1 hat auch der ihr zugeordnete dreidimensionale interessierende Bereich (interessierendes Volumen) eine definierte Position im raumfesten oder patientenfesten Koordinatensystem x, y, z. Das Baryzentrum des interessierenden Bereiches 3 kann im Bereich der darzustellenden anatomischen Patientenstruktur platziert werden (beispielsweise auf einem Teil eines Gefäßes), und es wird auf dieser Position verbleiben.

Die obige Situation kann anhand der Figur 2 verdeutlicht werden. Dort ist in der linken Abbildung zunächst der Ausgangszustand dargestellt, wo die Schnittfläche 4 der Bilderfassungsebene 2 mit dem interessierenden Bereich über den Gefäßen 6, 7 und 8 liegt. Wenn nun die Sonde 1 bewegt wird, würde sich die Schnittfläche 4 von den Gefäßen 6, 7, 8 weg verschieben, wie in der rechten Darstellung in der Figur 2 mit dem größer schraffierten Bereich 4 aufgezeigt. Weil die Bewegung der Sonde aber durch die Referenzanordnung 10 verfolgt und quantifiziert werden kann, kann der interessierende Bereich entsprechend zurück verschoben werden, so dass seine Schnittfläche 9 mit der Erfassungsebene 2 wieder im Bereich der Gefäße liegt. Die Einstellungen für den interessierenden Bereich in der Ultraschall-Hardware/Software verfolgen also die Bewegung und verschieben den interessierenden Bereich wieder auf die darzustellenden Patientenstrukturen (Blutgefäße). Optional kann das Zentrum des interessierenden Volumens 3 automatisch entlang einer Patientenstruktur (beispielsweise eines segmentierten Blutgefäßes) oder anhand jedweder anderer Information aus vorab akquirierten Daten (CT, MRI, ...) automatisch gesetzt werden, so dass das Zentrum des interessierenden Bereichs des Ultraschallgerätes immer an der aktuellen Bildposition der Gefäßstruktur gehalten wird. Das Zentrum des interessierenden Volumens kann also entweder manuell vor oder während der Untersuchung markiert werden (Landmarkenpunkt), oder automatisch mittels einer segmentierten Patientenstruktur erfasst werden, welche in der Ultraschall-Erfassungsebene 2 liegt.

Mit anderen Worten verwendet das erfindungsgemäße Verfahren die Information aus dem Navigationssystem, um die Koordinaten bzw. neue Koordinaten eines ausgewählten interessierenden Bereiches in einem raumfesten oder patientenfesten Koordinatensystem zu errechnen, wobei - wie oben beschrieben - die Kalibrierungsinformation der navigierten Ultraschallsonde verwendet wird. Diese raumfesten oder patientenfesten Koordinaten (Weltkoordinaten) sind dann bezüglich der Ausrichtung des Patienten "fixiert" und definieren auch das Zentrum des interessierenden Volumens. Dieser Bereich bzw. dieses Volumen ist beispielsweise eine kastenartige oder anders ausgebildete dreidimensionale Form, und das Baryzentrum dieser dreidimensionalen Form wird an der errechneten Position im Patienten-Koordinatensystem fixiert. Durch das Bewegen der navigierten Sonde im Patientenraum werden die aktuellen Einstellungen für den interessierenden Bereich der Sonde verändert, und zwar mit Hilfe der Information über die Schnittfläche der Bilderfassungsebene mit dem "fixierten" interessierenden Volumen. Deshalb wird beispielsweise bei der Ultraschallbildgebung ein anfangs ausgewähltes Blutgefäß immer im interessierenden Bereich bleiben, solange es irgendwo im Ultraschallbild sichtbar ist.

Die Form des interessierenden Bereiches bzw. des interessierenden Volumens kann für einige Einsatzfälle angepasst werden. Er bzw. es könnte beispielsweise eine größere Tiefe haben, um einer darzustellenden anatomischen Struktur besser folgen zu können. Das interessierende Volumen kann alternativ definiert werden, indem man einer bestimmten anatomischen Struktur folgt, beispielsweise einem Blutgefäß. In diesem Fall wird der interessierende Bereich des Ultraschallbildes anhand des Schnittpunktes der Gefäßstruktur mit der Bilderfassungsebene ausgewählt, wodurch ein Bereich um den Punkt herum selektiert wird, wo das Gefäß im Bild sichtbar ist. Der Schnittpunkt des Gefäßes mit der Bilderfassungsebene kann beispielsweise durch ein segmentiertes Objekt aus einer Gefäßerkennung im vorab akquirierten Bilddatensatz definiert werden, oder durch jedwedes andere Objekt aus der voroperativen Behandlungsplanung.

Gerade im Fall einer Doppler-Bildgebung mit einer Ultraschallvorrichtung ist die vorliegende Erfindung von Vorteil, wenn der Winkel des sich bewegenden Fluids bezüglich des Schallstrahls durch den Verwender korrekt berücksichtigt werden muss, um die Strömungsgeschwindigkeit richtig zu erfassen. Wenn ein vordefiniertes Gefäßobjekt verwendet wird, um den aktuell interessierenden Bereich einzustellen, kann das Verfahren den genannten Winkel durch die Schnittgeometrie des Gefäßes in der Bilderfassungsebene definieren und diese Information in der Ultraschallhardware bzw. -software einstellen. Dadurch kann eine korrekte Geschwindigkeitsanzeige erzielt werden, ohne dass ein weiterer Benutzereingriff notwendig wird.

Insgesamt ermöglicht es die Erfindung, dass der Verwender sich auf die Untersuchung, also die Darstellung konzentrieren kann, während er das Bilderfassungsgerät (Sonde) frei bewegt. Er muss die Bewegungen des Bilderfassungsgerätes nicht mehr räumlich einschränken, um die richtige Position des interessierenden Bereiches (zum Beispiel des Strömungsfensters) sicherzustellen, und er muss auch den interessierenden Bereich nicht jedes Mal anpassen, wenn er die Position oder den Winkel des Bilderfassungsgeräts ändert. Außerdem gestattet es das erfindungsgemäße Verfahren dem Verwender, einen relativ kleinen interessierenden Bereich einzustellen, was eine hohe Bildwiederholungsfrequenz und eine bessere und schnellere Ultraschall-Bilderfassung ermöglicht.

## Patentansprüche

1. Verfahren zur Verfolgung eines interessierenden Bereichs und zur Darstellung einer anatomischen Patienterstruktur im interessierenden Bereich (3) eines beweglichen Bilderfassungsgeräts (1), mit den folgenden Schritten:
- in einem raumfesten oder patientenfesten Koordinatensystem (x, y, z) werden Koordinaten der darzustellenden Patientenstruktur (6, 7, 8), die in einem Bilddatensatz des Patienten enthalten ist, bestimmt;
- in einem Koordinatensystem (u, v, w), das relativ zum beweglichen Bilderfassungsgerät (1) fest ist, wird ein interessierender Bereich (3) im Erfassungsbereich des Bilderfassungsgeräts (1) bestimmt, der die Patientenstruktur (6, 7, 8) umfasst und in welchem das Bilderfassungsgerät besondere Eigenschaften der Patientenstruktur (6, 7, 8) ermitteln kann;
- der interessierende Bereich wird an einer definierten Position im raumfesten oder patientenfesten Koordinatensystem (x, y, z) fixiert;
- die Veränderung der relativen Position von Bilderfassungsgerät (1) und Patientenstruktur (6, 7, 8) im raumfesten oder patientenfesten Koordinatensystem (x,
y, z) wird mittels eines medizintechnischen Tracking- bzw. Navigationssystems verfolgt;
- im Koordinatensystem (u, v, w) des Bilderfassungsgeräts (1) wird der interessierende Bereich (3) entsprechend der Bewegung des Bilderfassungsgeräts (1) mittels des im raumfesten oder patienten festen Koordinatensystems (x,y,z) fixierten interessierenden Bereichs nachgeführt, so dass dieser während und nach der Bewegung des Geräts an der Stelle der Patientenstruktur (6, 7, 8) gehalten wird und diese umfasst ; und
- die Patientenstruktur (6, 7, 8) wird mittels des Bilderfassungsgeräts und einer Bildausgabe dargestellt.

2. Verfahren nach Anspruch 1, bei dem der interessierende Bereich (3) ein interessierendes Volumen im Erfassungsbereich des Bilderfassungsgeräts (1) ist und dem interessierenden Bereich eine definierte Position im raumfesten oder patientenfesten Koordinatensystem (x, y, z) zugeordnet wird.

3. Verfahren nach Anspruch 1 oder 2, bei dem der interessierende Bereich des Bilderfassungsgeräts (1) über dessen Softwareunterstützung vor der Benutzung, anfangs oder während der Darstellung manuell bzw. durch einen Benutzer festgelegt wird.

4. Verfahren nach einem der Ansprüche 1 bis 3, bei dem das bewegliche Bilddarstellungsgerät während der Bilddarstellung bewegt wird, wobei der interessierende Bereich des Bilderfassungsgeräts, insbesondere durch Einstellen der Geräteparameter, entsprechend der Bewegung des Bilddarstellungsgeräts nachgeführt wird.

5. Verfahren nach einem der Ansprüche 1 bis 4, bei dem der interessierende Bereich des Bilderfassungsgeräts (1) über dessen Softwareunterstützung anfangs oder während der Darstellung automatisch und softwareseitig bei einem darzustellenden Abschnitt mit der Patientenstruktur (6, 7, 8) festgelegt wird.

6. Verfahren nach einem der Ansprüche 1 bis 5, bei dem ein darzustellender Abschnitt mit der Patientenstruktur (6, 7, 8) im raumfesten oder patientenfesten Koordinatensystem (x, y, z) während der Bilddarstellung verschoben wird, insbesondere entlang der Patientenstruktur (6, 7, 8) verschoben wird, wobei der interessierende Bereich des Bilderfassungsgeräts entsprechend der Bewegung des darzustellenden Anschnitts nachgeführt wird.

7. Verfahren nach einem der Ansprüche 1 bis 6, bei dem die Größe und/oder die Form des interessierenden Bereichs verändert, insbesondere an die Patientenstruktur angepasst werden.

8. Verfahren nach einem der Ansprüche 1 bis 7, bei dem als Bilderfassungsgerät ein Ultraschall-Bilderfassungsgerät (1) verwendet wird, insbesondere ein Doppler-Ultraschallgerät zur Erfassung von Strömungseigenschaften, speziell Strömungsgeschwindigkeiten, in durchströmten Patientengefäßen, speziell Blutgefäßen.

9. Verfahren nach Anspruch 8, bei dem die Winkellage des durchströmten Gefäßes zur Bilderfassungsebene des Ultraschall-Bilderfassungsgeräts (1) aus der Schnittgeometrie des Schnittbildes des Gefäßes bestimmt wird und die ermittelten Daten über die Strömungseigenschaften, insbesondere die Strömungsgeschwindigkeit, entsprechend des Winkels korrigiert werden.

## Claims

1. A method for tracking a region of interest and displaying an anatomical patient structure in the region of interest (3) of a movable image detection apparatus (1), comprising the following steps:
- co-ordinates of the patient structure (6, 7, 8) to be displayed, which is contained in an image data set of the patient, are determined in a co-ordinate system (x, y, z) which is spatially fixed or fixed relative to the patient;
- a region of interest (3) within the detection range of the image detection apparatus (1), which includes the patient structure (6, 7, 8) and in which the image detection apparatus can ascertain particular properties of the patient structure (6, 7, 8), is determined in a co-ordinate system (u, v, w) which is fixed relative to the movable image detection apparatus (1);
- the region of interest is fixed at a defined position in the co-ordinate system (x, y, z) which is spatially fixed or fixed relative to the patient;
- the change in the relative position between the image detection apparatus (1) and the patient structure (6, 7, 8) in the co-ordinate system (x, y, z) which is spatially fixed or fixed relative to the patient is tracked by means of a medical tracking and/or navigation system;
- in the co-ordinate system (u, v, w) of the image detection apparatus (1), the region of interest (3) is guided in accordance with the movement of the image detection apparatus (1) by means of the region of interest which is fixed in the co-ordinate system (x, y, z) which is spatially fixed or fixed relative to the patient, such that the region of interest (3) is kept at the location of the patient structure (6, 7, 8) and includes it during and after the movement of the apparatus; and
- the patient structure (6, 7, 8) is displayed by means of the image detection apparatus and an image output.

2. The method according to claim 1, wherein the region of interest (3) is a volume of interest within the detection range of the image detection apparatus (1), and the region of interest is assigned a defined position within the co-ordinate system (x, y, z) which is spatially fixed or fixed relative to the patient.

3. The method according to claim 1 or 2, wherein the region of interest of the image detection apparatus (1) is defined via its software support before being used, at the beginning of or during display, manually and/or by a user.

4. The method according to any one of claims 1 to 3, wherein the movable image detection apparatus is moved while the image is being displayed, wherein the region of interest of the image detection apparatus is guided in accordance with the movement of the image detection apparatus, in particular by setting the apparatus parameters.

5. The method according to any one of claims 1 to 4, wherein the region of interest of the image detection apparatus (1) is defined via its software support, at the beginning or during display, automatically at the software end, and in a section which is to be displayed and includes the patient structure (6, 7, 8).

6. The method according to any one of claims 1 to 5, wherein a section which is to be displayed and includes the patient structure (6, 7, 8) is shifted within the co-ordinate system (x, y, z) which is spatially fixed or fixed relative to the patient, while the image is being displayed, in particular along the patient structure (6, 7, 8), wherein the region of interest of the image detection apparatus is guided in accordance with the movement of the section to be displayed.

7. The method according to any one of claims 1 to 6, wherein the size and/or shape of the region of interest is changed and in particular adjusted to the patient structure.

8. The method according to any one of claims 1 to 7, wherein an ultrasound image detection apparatus (1) is used as the image detection apparatus, in particular a Doppler ultrasound apparatus for detecting flow properties, specifically flow velocities, in through-flow patient vessels, specifically blood vessels.

9. The method according to claim 8, wherein the angular position of the through-flow vessel relative to the image detection plane of the ultrasound image detection apparatus (1) is determined from the sectional geometry of the sectional image of the vessel, and the ascertained data concerning the flow properties, in particular the flow velocity, are corrected in accordance with the angle.

## Revendications

1. Procédé pour suivre une région d'intérêt et pour afficher une structure anatomique d'un patient dans la région d'intérêt (3) d'un appareil mobile de détection d'image (1), comportant les étapes suivantes consistant à :
- dans un système de coordonnées (x, y, z) spatialement fixe ou fixe par rapport au patient, déterminer des coordonnées de la structure de patient à afficher (6, 7, 8), ladite structure étant contenue dans un ensemble de données d'image du patient,
- dans un système de coordonnées (u, v, w) qui est fixe par rapport à l'appareil mobile de détection d'image (1), déterminer une région d'intérêt (3) dans la région de détection de l'appareil de détection d'image (1), ladite région de détection comportant la structure de patient (6, 7, 8), et dans lequel l'appareil de détection d'image peut déterminer des propriétés particulières de la structure de patient (6, 7, 8),
- fixer la région d'intérêt dans une position définie dans le système de coordonnées spatialement fixe ou fixe par rapport au patient (x, y, z),
- changer la position relative de l'appareil de détection d'image (1) et de la structure de patient (6, 7, 8) dans le système de coordonnées spatialement fixe ou fixe par rapport au patient (x, y, z) au moyen d'un système de repérage ou de navigation médico-technique,
- dans le système de coordonnées (u, v, w) de l'appareil de détection d'image (1), suivre la région d'intérêt (3) correspondant au déplacement de l'appareil de détection d'image (1) au moyen de la région d'intérêt fixée dans le système de coordonnées spatialement fixe ou fixe par rapport au patient (x, y, z), de telle sorte que celle-ci est maintenue à l'emplacement de la structure de patient (6, 7, 8) pendant et après le déplacement de l'appareil et inclut celle-ci, et
- afficher la structure de patient (6, 7, 8) au moyen de l'appareil de détection d'image et d'une sortie d'image.

2. Procédé selon la revendication 1, dans lequel la région d'intérêt (3) est un volume d'intérêt dans la région de détection de l'appareil de détection d'image (1), et une position définie dans le système de coordonnées spatialement fixe ou fixe par rapport au patient (x, y, z) est affectée à la région d'intérêt.

3. Procédé selon la revendication 1 ou 2, dans lequel la région d'intérêt de l'appareil de détection d'image (1) est définie manuellement ou par un utilisateur à l'aide de moyens logiciels avant l'utilisation, au début ou pendant l'affichage.

4. Procédé selon l'une quelconque des revendications 1 à 3, dans lequel l'appareil mobile d'affichage d'image est déplacé pendant l'affichage d'image, dans lequel la région d'intérêt de l'appareil de détection d'image est suivie, en particulier en réglant les paramètres de l'appareil, en fonction du déplacement de l'appareil d'affichage d'image.

5. Procédé selon l'une quelconque des revendications 1 à 4, dans lequel la région d'intérêt de l'appareil de détection d'image (1) est définie à l'aide de moyens logiciels, au début ou pendant l'affichage, automatiquement et côté logiciel par une section à afficher avec la structure de patient (6, 7, 8).

6. Procédé selon l'une quelconque des revendications 1 à 5, dans lequel une section à afficher avec la structure de patient (6, 7, 8) dans le système de coordonnées spatialement fixe ou fixe par rapport au patient (x, y, z) est déplacée lors de l'affichage d'image, est en particulier déplacée le long de la structure de patient (6, 7, 8), la région d'intérêt de l'appareil de détection d'image étant suivie en fonction du déplacement de la section à afficher.

7. Procédé selon l'une quelconque des revendications 1 à 6, dans lequel la taille et/ou la forme de la région d'intérêt change, en particulier est adaptée à la structure de patient.

8. Procédé selon l'une quelconque des revendications 1 à 7, dans lequel un appareil de détection d'image à ultrasons (1) est utilisé comme appareil de détection, en particulier un appareil à ultrasons Doppler, pour détecter des propriétés d'écoulement, spécialement des vitesses d'écoulement, dans des vaisseaux de patient traversés par un écoulement, spécialement des vaisseaux sanguins.

9. Procédé selon la revendication 8, dans lequel la position angulaire du vaisseau traversé par un écoulement par rapport au plan de détection d'image de l'appareil de détection d'image par ultrasons (1) est déterminée à partir de la géométrie de section de l'image de section du vaisseau, et les données déterminées sont corrigées en fonction de l'angle en utilisant les propriétés d'écoulement, en particulier la vitesse d'écoulement.
